(19) Europäisches Patentamt / European Patent Office / Office européen des brevets

(11) **EP 2 590 701 B1**

(12) **FASCICULE DE BREVET EUROPEEN**

(45) Date de publication et mention
de la délivrance du brevet:
**18.03.2015 Bulletin 2015/12**

(21) Numéro de dépôt: **11743306.0**

(22) Date de dépôt: **06.07.2011**

(51) Int Cl.:
*A61M 16/00* (2006.01)          *A61B 5/08* (2006.01)
*A61B 5/048* (2006.01)          *G06K 9/00* (2006.01)

(86) Numéro de dépôt international:
**PCT/FR2011/051611**

(87) Numéro de publication internationale:
**WO 2012/004534 (12.01.2012 Gazette 2012/02)**

(54) **SYSTÈME DE DÉTECTION ÉLECTROENCÉPHALOGRAPHIQUE D'UNE INADÉQUATION ENTRE L'ÉTAT D'UN PATIENT PLACÉ SOUS ASSISTANCE VENTILATOIRE ET LE RÉGLAGE DE LA MACHINE UTILISÉE POUR CETTE ASSISTANCE, ET UTILISATION DE CETTE DÉTECTION POUR L'ADAPTATION DU RÉGLAGE**

SYSTEM ZUR EEG-ERKENNUNG EINES KONFLIKTS ZWISCHEN DEM ZUSTAND EINES UNTER BEATMUNGSUNTERSTÜTZUNG STEHENDEN PATIENTEN UND DER STEUERUNG DER FÜR DIESE UNTERSTÜTZUNG VERWENDETEN MASCHINE SOWIE VERWENDUNG DIESER ERKENNUNG ZUR EINSTELLUNG DER STEUERUNG

SYSTEM FOR ELECTROENCEPHALOGRAPHIC DETECTION OF AN INADEQUATENESS BETWEEN THE STATE OF A PATIENT PLACED UNDER RESPIRATORY ASSISTANCE AND THE CONTROL OF THE MACHINE USED FOR THIS ASSISTANCE, AND USE OF THIS DETECTION TO ADJUST THE CONTROL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.07.2010 FR 1055515**

(43) Date de publication de la demande:
**15.05.2013 Bulletin 2013/20**

(73) Titulaires:
• **Université Pierre et Marie Curie (Paris 6)**
  **75005 Paris (FR)**
• **Assistance Publique-Hôpitaux de Paris (APHP)**
  **75004 Paris (FR)**
• **UNIVERSITE DE LILLE 2**
  **59800 Lille (FR)**
• **Centre Hospitalier Régional et Universitaire de Lille**
  **59800 Lille (FR)**

(72) Inventeurs:
• **SIMILOWSKI, Thomas**
  **92130 Issy Les Moulineaux (FR)**

• **RAUX, Mathieu**
  **75014 Paris (FR)**
• **TYVAERT, Louise**
  **59800 Lille (FR)**

(74) Mandataire: **Blot, Philippe Robert Emile**
  **Cabinet Lavoix**
  **2, place d'Estienne d'Orves**
  **75441 Paris Cedex 09 (FR)**

(56) Documents cités:
WO-A1-00/00245          FR-A1- 2 903 314
US-A- 5 520 192          US-A1- 2004 254 493
US-A1- 2007 038 382

• **K J BLINOWSKA: "Methods of localization of time-frequency specific activity and estimation of information transfer in brain", INTERNATIONAL JOURNAL OF BIOELECTROMAGNETISM, vol. 10, no. 1, 2008, pages 2-16, XP002627002,**

**Description**

La présente invention concerne un système de détection électroencéphalographique d'un réglage inapproprié d'une machine d'assistance ventilatoire utilisée sur un mammifère, comportant :

- un électroencéphalographe, propre à mesurer, en fonction du temps, un signal électroencéphalographique représentatif d'un processus respiratoire ; et
- une entrée pour la réception d'un signal de déclenchement respiratoire, différent du signal électroencéphalographique, propre à indiquer un instant de déclenchement respiratoire.

Certaines personnes souffrent d'une défaillance respiratoire aiguë, consécutive, par exemple, à une pneumonie, un oedème pulmonaire ou une surinfection de maladies respiratoires chroniques. Une assistance ventilatoire mécanique peut s'avérer nécessaire chez ces personnes. Les machines d'assistance ventilatoire ou ventilateurs comportent des moyens de détection d'une inspiration par le patient et des moyens pour aider le patient à l'inspiration en augmentant le flux d'air ou la pression de l'air recueilli par le patient. Les ventilateurs comportent également des moyens de détection de l'expiration et des moyens pour interrompre l'activité inspiratoire du ventilateur lorsque l'expiration est détectée, ce qui permet de synchroniser au mieux l'activité du patient et celle du ventilateur.

Ainsi, l'assistance consiste en la fourniture d'un volume prédéterminé de gaz ou en une pressurisation des voies aériennes. Dans les deux cas, divers réglages permettent d'adapter le flux de gaz aux besoins du patient. La machine d'assistance doit être adaptée au comportement ventilatoire du patient afin que la relation qui les unit soit "harmonieuse", c'est-à-dire que le patient soit dans un état de confort physique satisfaisant, le patient n'éprouvant, lors de l'assistance, aucune gêne respiratoire. Avec des réglages inadaptés, par exemple lorsque le flux d'air apporté est trop important ou, au contraire, trop faible, le patient peut être dans une situation inconfortable, voire être dans une situation de détresse respiratoire. Il en va de même lorsque l'activité inspiratoire du ventilateur se prolonge alors que le patient est en phase d'expiration. Il semble que de telles circonstances soient délétères pour le patient.

Pour détecter une telle dysharmonie entre le patient et la machine d'assistance ventilatoire, résultant d'un réglage inapproprié de la machine d'assistance ventilatoire, différents moyens ont été utilisés. En particulier, il est possible de simplement interroger le patient. Toutefois, cela n'est pas possible lorsque le patient est endormi ou dans le coma.

Il est également possible d'observer l'activité ventilatoire du patient, et notamment la fréquence et l'utilisation des différents groupes musculaires respiratoires.

Enfin, il est possible d'étudier des signaux de pression et de débit fournis par le ventilateur pour détecter la survenue d'événements témoignant d'une synchronisation imparfaite du ventilateur et du patient. Il existe divers profils témoignant de cette synchronisation imparfaite, comme par exemple les "appels inefficaces", au cours desquels le patient fait un effort inspiratoire qui n'est pas "récompensé" par le ventilateur.

En pratique, ces moyens s'avèrent délicats à utiliser et constituent tous des témoins indirects des sensations que peut éprouver le patient.

FR-A-2 903 314 décrit un procédé de détection d'une dysharmonie entre un patient et une machine d'assistance ventilatoire consistant, pour chaque cycle respiratoire, à mesurer un signal électroencéphalographique sur un intervalle de mesure s'étendant autour d'un instant de déclenchement respiratoire, puis à moyenner les signaux électroencéphalographiques mesurés sur plusieurs intervalles de mesure et enfin de traiter le signal moyenne ainsi obtenu pour en déduire une éventuelle dysharmonie entre le patient et la machine d'assistance ventilatoire.

Un tel procédé ne donne pas entière satisfaction. En effet, dans les situations d'inconfort ou de détresse respiratoire résultant d'une dysharmonie entre le patient et le ventilateur, il est important d'être en mesure de détecter cette dysharmonie et d'y remédier le plus rapidement possible pour rétablir l'harmonie entre le patient et son ventilateur.

Le procédé tel que décrit dans FR-A-2 903 314, nécessite de mesurer et de moyenner un signal électroencéphalographique sur au moins soixante à quatre-vingt cycles respiratoires pour être en mesure de conclure à l'existence d'une dysharmonie. Ceci correspond, en moyenne, à une durée de quatre à cinq minutes, pendant laquelle la dysharmonie détectée n'est par définition pas l'objet d'une correction, que celle-ci soit effectuée par un médecin alerté par la mise en évidence du signal électroencéphalographique anormal, ou par l'activation d'une boucle de régulation sur le ventilateur. Par ailleurs, le signal mesuré par le processus de moyennage, appelé potentiel prémoteur, peut facilement être parasité, soit par des mouvements du patient, soit par la pollution électromagnétique caractéristique des environnements de réanimation et de soins intensifs. Ainsi, il apparaît souhaitable d'améliorer à la fois la réactivité et la fiabilité de la détection électroencéphalographique de la dysharmonie patient-ventilateur.

Un but de l'invention est donc de proposer un système de détection électoencéphalographique d'un réglage inapproprié d'une machine d'assistance ventilatoire qui soit plus fiable, et en particulier qui permette une détection en temps réel d'une dysharmonie et donc d'un réglage inapproprié d'une machine d'assistance ventilatoire.

A cet effet, l'invention a pour objet un système de détection selon la revendication 1.

Selon des modes de réalisation particuliers, le système de détection d'un réglage inapproprié selon l'invention comporte l'une ou plusieurs des caractéristiques des revendications 2 à 7.

L'invention a également pour objet une installation d'assistance ventilatoire selon les revendications 8 et 9.

L'invention a également pour objet un procédé de détection d'un réglage inapproprié d'une machine d'assistance ventilatoire selon la revendication 10.

L'invention a également pour objet un produit programme d'ordinateur selon la revendication 11.

L'invention sera mieux comprise à la lecture de la description qui va suivre, donnée uniquement à titre d'exemple et faite en se référant aux dessins annexés, sur lesquels :

- la figure 1 est une vue schématique d'une installation d'assistance ventilatoire mise en oeuvre sur un patient ;
- la figure 2 est un exemple de courbes représentant des étapes de traitement d'un signal électroencéphalographique par le système de détection électroencéphalographique d'un réglage inapproprié selon l'invention ;
- la figure 3 est un exemple d'un ensemble de courbes, obtenues à partir d'électrodes placées dans des localisations différentes, et représentant l'évolution en fonction du temps d'un rapport de désynchronisation d'un signal électroencéphalographique recueilli pour un patient placé sous assistance ventilatoire, en présence d'une dysharmonie entre le patient et la machine d'assistance ventilatoire ;
- la figure 4 est une vue similaire à celle de la figure 3, en l'absence de dysharmonie entre le patient et la machine d'assistance ventilatoire ;
- la figure 5 est un exemple de courbe représentant l'évolution d'un signal électroencéphalographique représentatif d'un phénomène respiratoire en fonction du temps ; et
- la figure 6 est une représentation du signal électroencéphalographique de la figure 5 sous la forme d'une carte temps-fréquence.

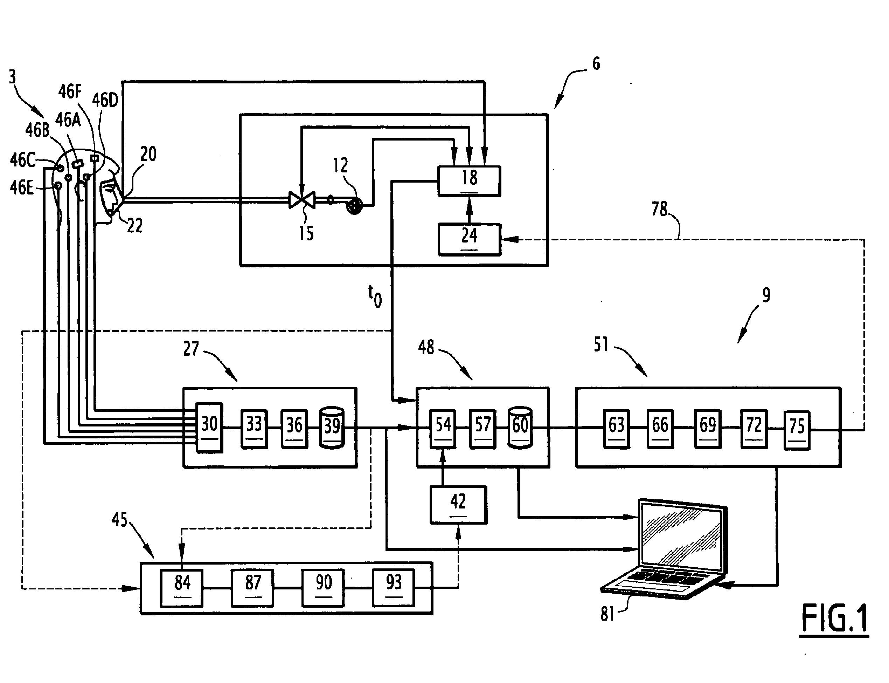

La figure 1 représente une installation 3 d'assistance ventilatoire utilisant un système de détection électroencéphalographique d'un réglage inapproprié selon l'invention.

Cette installation 3 comporte une machine d'assistance ventilatoire mécanique 6 et un système 9 de détection d'un réglage inapproprié de la machine d'assistance ventilatoire 6.

La machine 6 comporte, comme connu en soi, une turbine 12 propre à fournir un flux d'air à un patient à un débit déterminé et avec une pression donnée. En sortie de la turbine 12 est prévue une vanne 15 permettant de fournir ou non au patient l'air sous pression produit par la turbine 12. La turbine 12 et la vanne 15 sont reliées à une unité de pilotage 18 elle-même connectée à un capteur de dépression 20 propre à détecter une aspiration du patient.

La turbine 12 est reliée, en aval de la vanne 15, à un masque 22 propre à être appliqué sur les voies respiratoires supérieures du patient. Le capteur de dépression 20 est monté par exemple dans le masque 22 du patient.

En variante, le masque 22 peut être remplacé par une sonde endotrachéale.

L'unité de pilotage 18 est reliée à une unité de réglage 24 propre à modifier les paramètres de fonctionnement de la machine 6, et en particulier le débit imposé par la turbine 12, la pression du flux d'air, les instants de basculement de la vanne 15, et tout autre paramètre comme connu dans l'état de la technique. Par ailleurs, l'unité de pilotage 18 comporte une sortie propre à fournir un signal de déclenchement respiratoire $t_0$ représentatif d'un début d'inspiration du patient. Dans ce mode de réalisation, l'instant de déclenchement respiratoire $t_0$ est détecté par l'unité de pilotage 18 au moyen du capteur de dépression 20. Selon une variante, l'instant de déclenchement respiratoire $t_0$ correspond à un signal émis par la machine 6 lorsqu'elle commence à fournir de l'air au patient.

Dans la suite, on entend par cycle respiratoire l'intervalle de temps correspondant à une expiration suivie d'une inspiration complète. Chaque cycle respiratoire s'étend autour d'un instant de déclenchement respiratoire $t_0$.

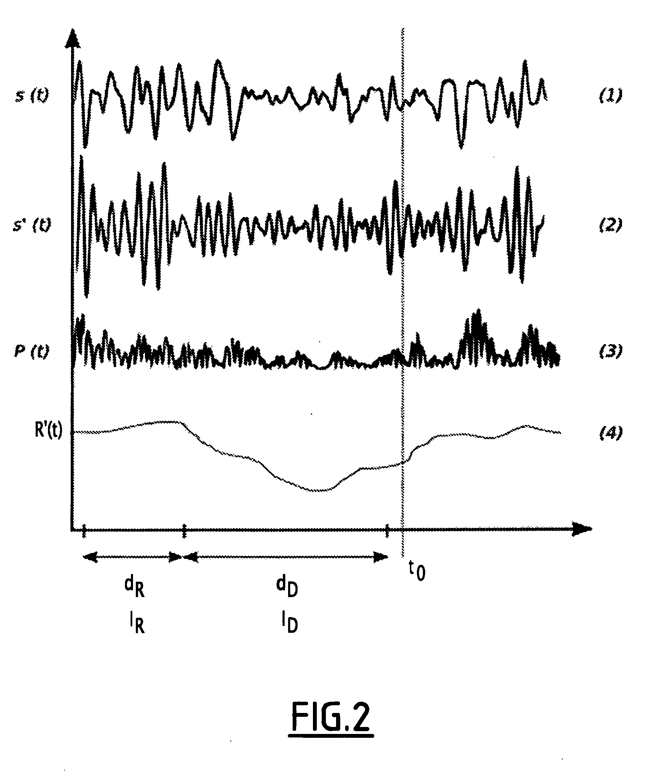

Le système 9 de détection d'un réglage inapproprié comporte un électroencéphalographe 27, propre à mesurer un signal électroencéphalographique (EEG) représentatif de la respiration, et propre à fournir un signal EEG s(t) en fonction du temps. Un tel signal s(t) est représenté par la courbe 1 de la figure 2.

L'électroencéphalographe 27 comporte par exemple entre 2 et 64 électrodes disposées sur le crâne du patient et notamment au niveau de l'aire motrice supplémentaire, c'est-à-dire du cortex prémoteur. Dans le mode de réalisation représenté, l'électroencéphalographe 27 comporte 6 électrodes 46A, 46B, 46C, 46D, 46E, 46F. Dans ce mode de réalisation, les électrodes 46E et 46F constituent respectivement une électrode de référence et une électrode de terre. La mise en oeuvre de six électrodes 46A, 46B, 46C, 46D, 46E, 46F constitue une configuration typique à l'utilisation en réanimation. Les électrodes 46A, 46B, 46C, 46D, 46E, 46F peuvent être de toute nature ; il s'agit notamment d'électrodes aiguille ou d'électrodes de surface.

Comme connu en soi, l'électroencéphalographe 27 comporte des moyens 30 de recueil du signal électroencéphalographique s(t), des moyens 33 de filtrage et d'amplification, propre à filtrer le signal électroencéphalographique s(t) mesuré, par exemple dans une bande de fréquence comprise entre 0,03 et 40 Hz ainsi que des moyens 36 d'échantillonnage afin de numériser le signal EEG s(t), par exemple avec une fréquence d'échantillonnage de 256 Hz. Il comporte en outre des moyens informatiques 39 de stockage des valeurs échantillonnées, associées à leur instant d'échantillonnage respectif.

Le système 9 comporte également des moyens de spécification 42 d'une bande de fréquence bêta, comprise entre 15 et 30 Hz et d'une largeur comprise entre 5 et 10 Hz. Cette bande de fréquence bêta correspond à la bande de fréquence de l'EEG, dans laquelle on observe, lors de l'exécution motrice d'une commande corticale, une diminution de la puissance du signal EEG avant l'instant de début d'exécution du mouvement, suivie d'une augmentation de la puissance du signal EEG, après l'instant de début d'exécution du mouvement. Cette diminution de la puissance correspond au phénomène de désynchronisation liée à l'évènement, tandis que l'augmentation de la puissance correspond au phénomène de synchronisation liée au mouvement. La bande de fréquence bêta varie en fonction des patients.

Les moyens de spécification 42 comportent par exemple une interface informatique de saisie, configurée pour recevoir des valeurs de borne inférieure et de borne supérieure de la bande de fréquence bêta, saisies par un utilisateur.

En variante, les moyens de spécification 42 sont connectés à la sortie de moyens 45 de détermination de la bande de fréquence bêta, tels que décrits plus loin.

Le système 9 comporte en outre une unité de traitement 48 du signal électroencéphalographique s(t) mesuré, connectée en entrée à l'électroencéphalographe 27 pour recevoir le signal électroencéphalographique s(t) fourni par l'électroencéphalographe 27. L'unité de traitement 48 comporte une entrée pour la réception du signal de déclenchement respiratoire $t_0$, connectée à la sortie correspondante de la machine 6, ainsi qu'une entrée pour la réception de la bande de fréquence bêta, connectée à la sortie des moyens de spécification 42.

A sa sortie, l'unité de traitement 48 est connectée à des moyens 51 de mise en évidence d'un réglage inapproprié de la machine d'assistance ventilatoire 6.

L'unité de traitement 48 est propre à traiter le signal électroencéphalographique s(t) fourni par l'électroencéphalographe 27 dans la seule bande de fréquence bêta spécifiée par l'intermédiaire des moyens de spécification 42, au fur et à mesure de l'acquisition de ce signal s(t) par l'électroencéphalographe 27.

L'unité de traitement 48 comprend des moyens de filtrage 54, propres à filtrer le signal EEG s(t) fourni par l'électroencéphalographe 27, dans la bande de fréquence bêta spécifiée par les moyens de spécification 42, de manière à ne conserver que les composantes du signal EEG s(t) de fréquence comprise dans la bande de fréquence bêta. Les moyens de filtrage 54 comprennent par exemple un filtre à réponse impulsionnelle infinie d'ordre 6. Les moyens de filtrage 54 sont propres à fournir, en sortie, un signal EEG s'(t) dans la seule bande de fréquence bêta, dont un exemple est représenté par la courbe 2 de la figure 2.

Les moyens de filtrage 54 sont connectés en sortie à des moyens de calcul 57, propres à calculer la puissance instantanée P(t) du signal EEG s'(t) dans la seule bande de fréquence bêta. A cet effet, les moyens de calcul 57 sont propres à calculer le module au carré de l'amplitude du signal électroencéphalographique s'(t) en appliquant la formule suivante: $P(t)=|s'(t)|^2$. La courbe 3 de la figure 2 représente un exemple de la puissance instantanée P(t) ainsi calculée.

Selon un premier mode de réalisation, les moyens de calcul 57 sont en outre configurés pour calculer, pour chaque cycle respiratoire comprenant un instant de déclenchement respiratoire $t_0$, la puissance moyenne $P_{mD}$ du signal EEG s'(t) dans la seule bande de fréquence bêta, sur un intervalle $I_D$, de durée $d_D$, s'étendant en avance de l'instant de déclenchement respiratoire $t_0$, ainsi que la puissance moyenne $P_{mR}$ de ce signal s'(t) sur un intervalle $I_R$, de durée $d_R$, s'étendant en avance de l'intervalle $I_D$.

Le début de l'intervalle $I_D$ est antérieur à l'instant de déclenchement respiratoire $t_0$ de moins de 2 secondes. Le début de l'intervalle $I_R$ est antérieur à l'instant de déclenchement respiratoire $t_0$ de 3,5 à 1 secondes.

La durée $d_D$ de l'intervalle $I_D$ est comprise entre 0,125 et 2 secondes. L'intervalle $I_D$ s'étend entièrement avant l'instant de déclenchement respiratoire $t_0$. La durée $d_R$ de l'intervalle $I_R$ est comprise entre 0,1 et 0,5 secondes. L'intervalle $I_R$ s'étend en avance de l'intervalle $I_D$ et ne chevauche pas l'intervalle $I_D$.

Pour calculer la puissance moyenne $P_{mD}$ du signal s'(t) sur l'intervalle $I_D$, les moyens de calcul 57 sont configurés pour appliquer, par exemple, la formule (1) suivante :

$$P_{mD} = \frac{1}{t_2 - t_1} \int_{t=t_1}^{t=t_2} P(t)dt \qquad (1)$$

où
$t_1$ correspond à l'instant de début de l'intervalle $I_D$ ;
$t_2 = t_1 + d_D$ ; et
P(t) est la puissance instantanée du signal EEG s'(t) dans la seule bande de fréquence bêta, calculée par les moyens de calcul 57.

Pour calculer la puissance moyenne $P_{mR}$ du signal s'(t) sur l'intervalle $I_R$, les moyens de calcul 57 sont configurés pour appliquer la formule (1), $t_1$ correspondant à l'instant de début de l'intervalle $I_R$ et $t_2 = t_1 + d_R$.

L'unité de traitement 48 comprend en outre des moyens de stockage 60, propres à stocker la puissance instantanée P(t), ainsi que les puissances moyennes $P_{mD}$ et $P_{mR}$ calculées par les moyens de calcul 57.

Les moyens de mise en évidence 51 d'un réglage inapproprié de la machine 6 sont propres à mettre en évidence, pour chaque cycle respiratoire, une éventuelle désynchronisation du signal EEG s'(t) dans la bande de fréquence bêta spécifiée.

Les moyens de mise en évidence 51 comprennent des moyens de comparaison 63, configurés pour comparer, pour chaque cycle respiratoire, la puissance moyenne $P_{mD}$ calculée sur l'intervalle $I_D$ à la puissance moyenne $P_{mR}$, calculée sur l'intervalle $I_R$. A cet effet, ils sont par exemple configurés pour calculer, pour chaque cycle respiratoire, un rapport de désynchronisation moyen R, représentatif d'une désynchronisation du signal EEG s'(t) dans la bande de fréquence bêta, en appliquant la formule suivante :

$$ R = \frac{P_{mD} - P_{mR}}{P_{mR}} \times 100 $$

où
$P_{mD}$ est la puissance moyenne du signal s'(t) sur l'intervalle $I_D$ ; et
$P_{mR}$ est la puissance moyenne du signal s'(t) sur l'intervalle $I_R$.

Les moyens de mise en évidence 51 comprennent en outre des moyens de détection 66, propres à détecter, pour chaque cycle respiratoire, s'il existe une désynchronisation du signal EEG s'(t). Les moyens de détection 66 sont propres à comparer, pour chaque cycle respiratoire, le rapport de désynchronisation moyen R à une valeur prédéterminée notée V. La valeur prédéterminée V est par exemple égale à -15%. Si les moyens de détection 66 détectent un rapport de désynchronisation moyen R inférieur à la valeur V prédéterminée, un compteur 69 du système de détection 9 est incrémenté.

Si les moyens de détection 66 détectent que le rapport de désynchronisation moyen R n'est pas inférieur à la valeur V prédéterminée, le compteur 69 est mis à zéro.

Selon une première variante, les moyens de calcul 57 de l'unité de traitement 48 sont propres à calculer la puissance moyenne $P_{mR}$ sur l'intervalle $I_R$ de la manière décrite ci-dessus et les moyens de comparaison 63 des moyens de mise en évidence 51 sont configurés pour calculer, sur l'intervalle $I_D$, un rapport de désynchronisation instantané R'(t) en appliquant la formule suivante :

$$ R'(t) = \frac{P(t) - P_{mR}}{P_{mR}} \times 100 $$

où
P(t) est la puissance instantanée du signal s'(t) sur l'intervalle $t_D$ et
$P_{mR}$ est la puissance moyenne du signal s'(t) sur l'intervalle $I_R$.

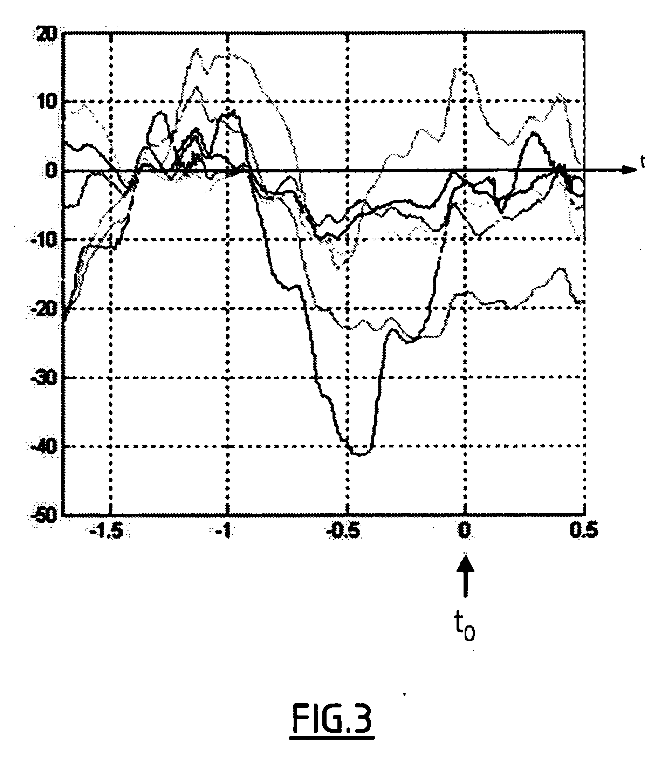

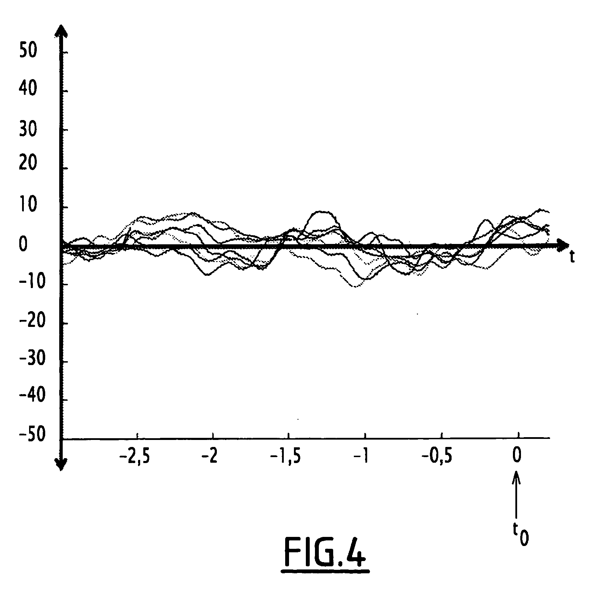

La courbe 4 de la figure 2 représente l'évolution du rapport de désynchronisation instantané R'(t) du signal s'(t) en fonction du temps. Les ensembles de courbes des figures 3 et 4 représentent l'évolution du rapport de désynchronisation instantané R'(t) du signal s'(t) en fonction du temps, respectivement en présence d'une désynchronisation et en l'absence d'une désynchronisation. Les courbes des figures 3 et 4 sont obtenues respectivement en appliquant les étapes de traitement indiquées ci-dessus au signal s(t) provenant d'électrodes placées au niveau de dérivations différentes. Les figures 3 et 4 ont été obtenues avec un nombre d'électrodes différent.

Selon cette première variante, les moyens de détection 66 sont configurés pour mettre en oeuvre, pour l'ensemble des valeurs du rapport de désynchronisation instantané R'(t), un test statistique, par exemple un test de Wilcoxon, pour déterminer, pour chaque cycle respiratoire, s'il existe un écart traduisant une désynchronisation entre la puissance P(t) du signal s'(t) dans l'intervalle $I_D$ et la puissance moyenne $P_{mR}$ du signal s'(t) dans l'intervalle $I_R$. Dans le cas où les moyens de détection 66 sont configurés pour mettre en oeuvre un test de Wilcoxon, l'hypothèse nulle est rejetée si le risque de première espèce est inférieur à 1%, c'est-à-dire qu'un écart traduisant une désynchronisation est constaté si le risque de première espèce est inférieur à 1%.

Selon une seconde variante, les moyens de calcul 57 de l'unité de traitement 48 sont configurés pour calculer la puissance moyenne $P_{mR}$ sur l'intervalle $I_R$ de la manière décrite ci-dessus et les moyens de détection 66 sont configurés pour mettre en oeuvre, pour l'ensemble des valeurs de la puissance instantanée P(t) calculées sur l'intervalle $I_D$, un test statistique, par exemple un test de Wilcoxon, pour déterminer, pour chaque cycle respiratoire, s'il existe un écart traduisant une désynchronisation entre la puissance P(t) du signal s'(t) dans l'intervalle $I_D$ et la puissance moyenne $P_{mR}$ du signal s'(t) dans l'intervalle $I_R$. Dans le cas où les moyens de détection 66 sont configurés pour mettre en oeuvre un test de Wilcoxon, l'hypothèse nulle est rejetée si le risque de première espèce est inférieur à 1%, c'est-à-dire qu'un écart traduisant une désynchronisation est constaté si le risque de première espèce est inférieur à 1%.

Selon ces première et deuxième variantes, à chaque cycle respiratoire, si les moyens de détection 66 détectent un écart traduisant une désynchronisation entre la puissance P(t) du signal s'(t) dans l'intervalle $I_D$ et la puissance moyenne $P_{mR}$ du signal s'(t) dans l'intervalle $I_R$, le compteur 69 est incrémenté.

Si les moyens de détection 66 ne détectent pas d'écart traduisant une désynchronisation entre la puissance P(t) du signal s'(t) dans l'intervalle $I_D$ et la puissance moyenne $P_{mR}$ du signal s'(t) dans l'intervalle $I_R$, le compteur 69 est mis à zéro.

Un signal EEG s(t) peut être obtenu en sortie de l'électroencéphalographe 27 pour chacune des électrodes de l'électroencéphalographe 27. Seul l'un de ces signaux s(t) a été représenté sur la figure 2. Le système de détection 9 selon l'invention est propre à appliquer le même traitement aux signaux EEG s(t) provenant de chacune des électrodes de l'électroencéphalographe 27, de manière à obtenir un rapport de désynchronisation moyen R ou un rapport de désynchronisation instantané R'(t) pour chacune d'elles.

Selon un mode de réalisation, seul l'un des signaux EEG s(t) provenant d'une électrode particulière est pris en compte pour l'incrémentation ou la mise à zéro du compteur 69 par les moyens de détection 66.

Selon une variante, les rapports de désynchronisation moyen R ou de désynchronisation instantané R' obtenus à partir de signaux EEG s(t) provenant de plusieurs électrodes sont pris en compte pour l'incrémentation ou la mise à zéro du compteur 69 par les moyens de détection 66.

Les moyens de mise en évidence 51 comprennent en outre des moyens 72 de déclenchement d'un indicateur, propres à déclencher un indicateur 75 lorsque le compteur 69 dépasse une valeur seuil prédéterminée, c'est-à-dire lorsqu'une désynchronisation a été détectée pendant un nombre prédéterminé de cycles respiratoires successifs.

L'indicateur 75 est ainsi propre à indiquer l'existence d'une dysharmonie entre le patient et la machine d'assistance 6 au praticien. L'indicateur 75 est par exemple un voyant lumineux, un signal sonore ou tout autre moyen adapté pour alarmer le praticien.

Selon une variante, les moyens de déclenchement 72 sont propres à déclencher l'indicateur 75 dès que le compteur 69 devient supérieur à 1, c'est-à-dire à chaque cycle respiratoire pour lequel les moyens de détection 66 détectent une désynchronisation.

Selon un mode de réalisation, une boucle de rétrocontrôle 78 relie le système de détection d'un réglage inapproprié 9 à la machine d'assistance 6. La boucle de rétrocontrôle 78 est propre à modifier les réglages de la machine d'assistance ventilatoire 6 lorsque le système de détection 9 détecte une dysharmonie, c'est-à-dire en particulier lorsque l'indicateur 75 est déclenché. A cet effet, la boucle de rétrocontrôle 78 est propre à mettre en oeuvre un algorithme de type connu, propre à modifier les réglages de la machine 6 par itérations successives, et propre à être mis en oeuvre uniquement lorsque le système 9 détecte une dysharmonie.

Le système 9 comporte en outre des moyens 81 de mise à disposition d'un praticien d'une information représentative d'un réglage inapproprié de la machine 6. Ces moyens 81 de mise à disposition sont reliés aux moyens 51 de mise en évidence. Ils comprennent par exemple un écran d'affichage et sont propres à afficher, par exemple, les courbes représentées sur la figure 2, en particulier l'évolution du rapport de désynchronisation instantané R'(t) au cours du temps et le signal EEG s(t) enregistré par l'électroencéphalographe 27, ainsi que le nombre de cycles respiratoires associés à une désynchronisation au cours des 20 derniers cycles respiratoires et le temps passé sans désynchronisation. Dans ce cas, les moyens 81 de mise à disposition sont également propres à recevoir des informations provenant de l'électroencéphalographe 27 et de l'unité de traitement 48.

En option, le système 9 de détection d'un réglage inapproprié comprend en outre, les moyens 45 de détermination de la bande de fréquence bêta. Ces moyens 45 de détermination comportent une entrée pour la réception du signal EEG s(t) fourni par l'électroencéphalographe 27 et une entrée pour la réception du signal de déclenchement respiratoire $t_0$, connectée à la sortie correspondante de la machine d'assistance 6.

Les moyens 45 de détermination de la bande de fréquence bêta comprennent des moyens 84 de segmentation du signal EEG s(t) en intervalles de temps $I_1$, $I_2$, ..., $I_n$ successifs identiques. Chaque intervalle de temps $I_1$, $I_2$, ..., $I_n$ comprend un instant de déclenchement respiratoire $t_0$. Chaque intervalle de temps $I_1$, $I_2$, ..., $I_n$ a une durée d. La durée d se compose d'une durée $d_1$ s'étendant en avance par rapport à l'instant de déclenchement respiratoire $t_0$ et d'une durée $d_2$ s'étendant en retard par rapport à l'instant de déclenchement respiratoire $t_0$. Les durées d, $d_1$ et $d_2$ sont identiques pour tous les intervalles $I_1$, $I_2$, ..., $I_n$.

La durée d est comprise entre 0,375 et 3 secondes, la durée $d_1$ comprise entre 0,125 et 2 secondes et la durée $d_2$ est comprise entre 0,250 et 1 seconde.

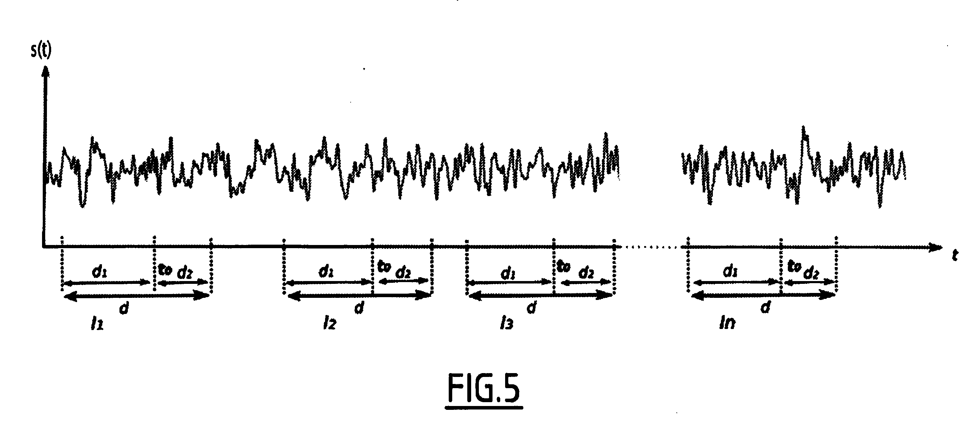

La figure 5 est une représentation schématique d'un signal EEG s(t) segmenté en intervalles $I_1$, $I_2$, ..., $I_n$ par les moyens de segmentation 84. Sur cette figure, la durée d est égale à 1,5 secondes, la durée $d_1$ à 1250 millisecondes et la durée $d_2$ à 250 millisecondes.

Les moyens 45 de détermination de la bande de fréquence bêta comprennent en outre des moyens 87 de rétro-moyennage des valeurs échantillonnées du signal EEG s(t) mémorisées sur n intervalles de temps $I_1$, $I_2$, ..., $I_n$ successifs, c'est-à-dire comportant des instants de déclenchement respiratoire $t_0$ successifs. Les moyens 87 de rétro-moyennage sont formés par exemple d'un micro-ordinateur mettant en oeuvre un programme adapté. Plus précisément, les moyens de rétro-moyennage 87 sont propres à effectuer la moyenne arithmétique point à point entre les valeurs échantillonnées correspondantes des n intervalles de temps successifs $I_1$, $I_2$ ...,$I_n$ du signal EEG enregistré. Le nombre n d'intervalles de temps $I_1$, $I_2$, ..., $I_n$ est compris entre 20 et 80, et est par exemple environ égal à 30.

Ainsi, les moyens de rétro-moyennage 87 sont configurés pour fournir, à partir des n portions de signal s(t) sur les intervalles $I_1$, $I_2$, ..., $I_n$, un signal moyen $s_m(t)$ sur un intervalle de temps $I_m$ ayant les mêmes caractéristiques que les intervalles $I_1$, $I_2$, ..., $I_n$ et comprenant donc un instant de déclenchement respiratoire $t_o$.

Les moyens 45 de détermination de la bande de fréquence bêta comprennent en outre des moyens 90 d'établissement d'une carte temps-fréquence, encore appelée spectrogramme, du signal moyen $s_m(t)$ obtenu en sortie des moyens de rétro-moyennage 87. La carte temps-fréquence est propre à indiquer l'évolution de la densité spectrale de puissance du signal moyen $s_m(t)$ en fonction de la fréquence dudit signal $s_m(t)$ et du temps.

Les moyens 90 d'établissement de la carte temps-fréquence sont par exemple configurés pour appliquer au signal moyen $s_m(t)$ une transformée de Fourier à court terme ou une transformée en ondelettes discrète pour obtenir un signal transformé F(f,t), puis pour calculer la densité spectrale de puissance P(f,t) du signal transformé F(f,t), cette densité spectrale de puissance P(f,t) correspondant au module au carré du signal transformé F(f,t). Les moyens 90 d'établissement de la carte temps-fréquence sont en outre configurés pour représenter la densité spectrale de puissance P(f,t) du signal transformé F(f,t) en fonction de la fréquence et du temps sur une carte temps-fréquence.

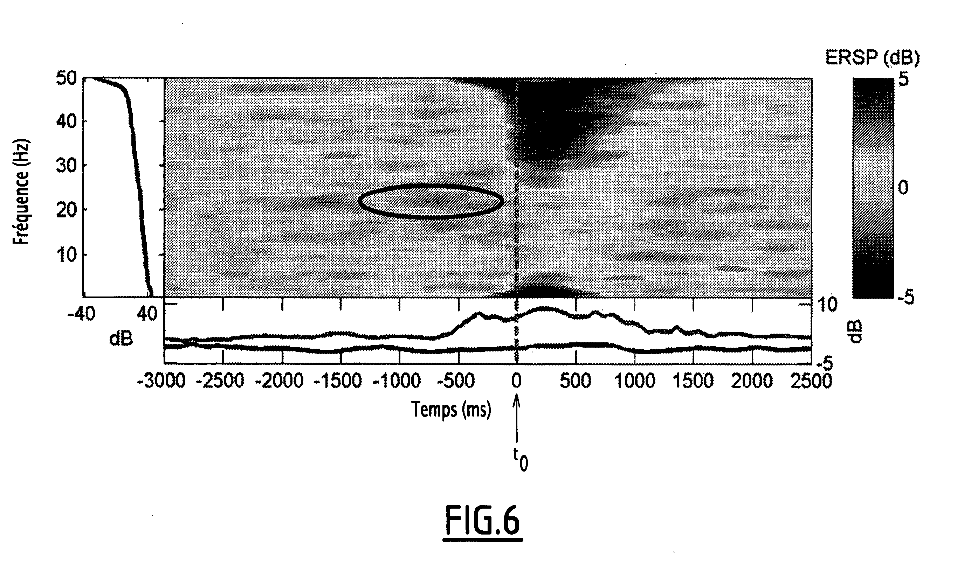

Selon un mode de réalisation, représenté sur la figure 6, la densité spectrale de puissance P(f,t) est exprimée en décibels, relativement à une densité spectrale de puissance de référence $P_{ref}$. Cette grandeur est notée ERSP (« Event related spectral perturbation » en anglais, et MSLE, i.e. « Modifications spectrales liées à l'événement » en français). La densité spectrale de puissance de référence $P_{ref}$ correspond à la valeur de la densité spectrale de puissance P(f,t) du signal transformé F(f,t) sur un intervalle de référence s'étendant en avance par rapport à l'instant de déclenchement respiratoire $t_0$. L'intervalle de référence a une durée comprise entre 150 et 300 ms. Le début de l'intervalle de référence est antérieur à l'instant de déclenchement respiratoire d'une durée comprise entre 0,1 et 0,5 secondes et par exemple d'environ 250 millisecondes. L'intervalle de référence s'étend en outre en avance par rapport à un intervalle de préparation du mouvement, c'est-à-dire de préparation de l'inspiration, et ne chevauche pas cet intervalle de préparation. Le début de l'intervalle de préparation est antérieur à l'instant de déclenchement respiratoire $t_0$ d'une durée comprise entre 0,1 et 0,5 secondes.

Enfin, les moyens de détermination 45 comprennent des moyens 93 de détection de la bande de fréquence bêta à partir de la carte temps-fréquence établie par les moyens 90 d'établissement de la carte temps-fréquence.

Les moyens 93 de détection de la bande de fréquence bêta sont propres à déterminer la bande de fréquence du signal EEG s(t) dans laquelle il existe, pendant une durée prédéterminée, un écart entre la densité spectrale de puissance P(f,t) du signal transformé F(f,t) et la densité spectrale de puissance de référence $P_{ref}$ supérieur à une valeur prédéterminée. Ainsi, les moyens de détection 93 sont propres à déterminer la bande de fréquence dans laquelle la densité spectrale de puissance P(f,t) du signal transformé F(f,t) est inférieure à la densité spectrale de puissance de référence $P_{ref}$ d'une valeur supérieure à la valeur prédéterminée. Cette valeur prédéterminée est par exemple environ égale à 15% et la durée prédéterminée est par exemple égale à 100 ms.

Dans l'exemple représenté sur la figure 6, les moyens de détection 93 sont propres à déterminer cet écart à partir de l'ERSP. Ils sont alors propres à déterminer la bande de fréquence dans laquelle, pendant la durée prédéterminée, la valeur de l'ERSP est inférieure à -0,7, ce qui correspond à une variation de 15% entre la densité spectrale de puissance P(f,t) et la densité spectrale de puissance de référence $P_{ref}$, la densité spectrale de puissance P(f,t) étant inférieure d'au moins 15% à la densité spectrale de puissance de référence $P_{ref}$. La bande de fréquence ainsi déterminée correspond à la bande de fréquence bêta. Elle est matérialisée par une ellipse sur la figure 6.

Comme cela a été indiqué ci-dessus, la bande de fréquence bêta correspond à une bande de fréquence du signal électroencéphalographique, dans laquelle une désynchronisation est observable avant le début du mouvement, matérialisé par l'instant de déclenchement respiratoire $t_0$. Une telle désynchronisation ne se produit que dans le cas de l'exécution d'une commande corticale. Lorsque le patient est en harmonie avec la machine 6, il n'y a pas d'activité corticale motrice liée à la respiration. Aucune désynchronisation n'est alors observée. La détermination de la bande de fréquence bêta par les moyens de détermination 45 nécessite donc que le patient fasse, pendant le nombre n de cycles respiratoires successifs, des mouvements respiratoires volontaires ou des mouvements respiratoires dont il est connu qu'ils répondent à une commande corticale.

Dans le cadre du procédé de détermination de la bande de fréquence bêta mis en oeuvre par les moyens 45 de détermination de la bande de fréquence bêta, une première méthode pour engendrer de tels mouvements respiratoires volontaires ou dont il est connu qu'ils répondent à une commande corticale consiste à volontairement dérégler la machine d'assistance 6 pendant un nombre n de cycles respiratoires, notamment si le patient est dans le coma. Ce déréglement consiste par exemple en une diminution transitoire de la sensibilité du seuil de déclenchement de l'assistance fournie par la machine d'assistance ventilatoire 6. En variante, si le patient est conscient et en mesure de respirer seul, il effectue un nombre n d'inspirations volontaires, par exemple sous la forme de reniflements volontaires maximaux.

Selon un mode de réalisation du procédé de détection d'un réglage inapproprié d'une machine d'assistance ventilatoire selon l'invention, la bande de fréquence bêta est déterminée une seule fois par les moyens 45 de détermination de la bande de fréquence bêta. Selon une variante, les moyens 45 de détermination de la bande de fréquence bêta déterminent la bande de fréquence bêta à intervalles réguliers pendant que le patient est ventilé artificiellement.

Le système de détection d'un réglage inapproprié d'une machine d'assistance ventilatoire selon l'invention présente l'avantage de ne pas nécessiter de moyennage du signal électroencéphalographique pour la détermination d'une dysharmonie. En effet, le système est en mesure de détecter l'existence d'une désynchronisation, et donc d'un effort respiratoire volontaire, ou plus généralement d'un effort respiratoire répondant à une commande corticale de la part du patient, signe d'une dysharmonie entre le patient et la machine, à chaque cycle respiratoire.

Ainsi, le système selon l'invention est propre à détecter une dysharmonie en temps réel, et d'y remédier aussi rapidement, en modifiant les réglages de la machine d'assistance ventilatoire, soit automatiquement par l'intermédiaire d'une boucle de rétrocontrôle, soit manuellement par un praticien.

En outre, le système selon l'invention est propre à analyser le signal de manière particulièrement fiable. En effet, le filtrage du signal électroencéphalographique dans la bande de fréquence bêta élimine un certain nombre d'artefacts du signal électroencéphalographique, sources d'erreurs, et notamment d'artefacts liés aux mouvements du patient, aux câbles de recueil du signal électroencéphalographique, à la présence d'un nombre important d'appareillages électriques (intervenant à la fréquence de 50 Hz en France et de 60 Hz aux Etats-Unis) ou d'artéfacts liés au phénomène de sudation du patient, responsable de variations du signal dans les basses fréquences, c'est-à-dire dans les fréquences inférieures à 1 Hz.

Enfin, le signal de désynchronisation, c'est-à-dire le signal représentant le rapport de désynchronisation instantané (courbe 4 de la figure 2) possède un rapport signal/bruit élevé, et notamment plus élevé que lorsque l'on utilise un signal obtenu par moyennage sans filtrage préalable dans la bande bêta.

**Revendications**

1. Système de détection (9) électroencéphalographique d'un réglage inapproprié d'une machine d'assistance ventilatoire (6) utilisée sur un mammifère, comportant :

   - un électroencéphalographe (27), propre à mesurer, en fonction du temps, un signal électroencéphalographique (s(t)) représentatif d'un processus respiratoire ; et
   - une entrée pour la réception d'un signal de déclenchement respiratoire, différent du signal électroencéphalographique (s(t)), propre à indiquer un instant de déclenchement respiratoire $(t_0)$;

   le système de détection (9) comportant en outre

   - des moyens de spécification (42) d'une bande de fréquence bêta comprise entre 15 et 30 Hz et de largeur comprise entre 5 et 10 Hz ;
   - des moyens de traitement (48) du signal électroencéphalographique mesuré, configurés pour traiter le signal électroencéphalographique mesuré, au fur et à mesure de son acquisition, dans la seule bande de fréquence bêta spécifiée ;
   - des moyens de mise en évidence (51), pour chaque cycle respiratoire, d'un réglage inapproprié de la machine d'assistance ventilatoire (6) à partir du signal électroencéphalographique traité dans la seule bande de fréquence bêta.

2. Système de détection (9) électroencéphalographique selon la revendication 1, **caractérisé en ce que** les moyens de mise en évidence (51) d'un réglage inapproprié sont configurés pour mettre en évidence, pour chaque cycle respiratoire, une éventuelle désynchronisation du signal électroencéphalographique dans la bande de fréquence bêta spécifiée, ladite désynchronisation précédant l'instant de déclenchement respiratoire $(t_0)$.

3. Système de détection (9) électroencéphalographique selon l'une des revendications 1 ou 2, **caractérisé en ce que** les moyens de traitement (48) comprennent en outre des moyens de calcul (57), configurés pour calculer la puissance instantanée (P(t)) dans la seule bande de fréquence bêta du signal électroencéphalographique, ainsi que des moyens de stockage (60) propres à stocker la puissance instantanée (P(t)) calculée.

4. Système de détection (9) électroencéphalographique selon la revendication 3, **caractérisé en ce que** les moyens de calcul (57) sont propres à calculer, pour chaque cycle respiratoire, à partir de la puissance instantanée (P(t)), une puissance moyenne $(P_{mR})$ du signal électroencéphalographique dans la seule bande de fréquence bêta sur un premier intervalle $(I_R)$, **en ce que** les moyens de mise en évidence (51) comprennent des moyens de comparaison (63) de la puissance instantanée (P(t)) calculée dans un second intervalle $(I_D)$ s'étendant en avance de l'instant de déclenchement respiratoire $(t_0)$ avec la puissance moyenne $(P_{mR})$ calculée sur le premier intervalle, le premier intervalle $(I_R)$ s'étendant en avance du deuxième intervalle $(I_D)$, et **en ce que** les moyens de mise en évidence (51) comprennent en outre des moyens de détection (66), propres à détecter, pour chaque cycle respiratoire, un écart entre la puissance moyenne $(P_{mR})$ calculée dans le premier intervalle $(I_R)$ et la puissance instantanée (P(t)) calculée dans le deuxième intervalle $(I_D)$.

5. Système de détection (9) électroencéphalographique selon la revendication 4, **caractérisé en ce que** les moyens de mise en évidence (51) comprennent des moyens de déclenchement (72) d'un indicateur, propres à déclencher un indicateur (75) si un écart est détecté pendant un nombre prédéterminé de cycles respiratoires successifs.

6. Système de détection (9) électroencéphalographique selon l'une quelconque des revendications précédentes, **caractérisé en ce qu'**il comprend en outre, en entrée des moyens de spécification (42) de la bande de fréquence bêta, des moyens de détermination (45) de la bande de fréquence bêta, lesdits moyens de détermination (45) de la bande de fréquence bêta comportant des moyens d'établissement d'une carte temps-fréquence (90) du signal électroencéphalographique mesuré par l'électroencéphalographe (27), ladite carte temps-fréquence étant propre à indiquer l'évolution de la densité spectrale de puissance du signal électroencéphalographique en fonction du temps et de la fréquence dudit signal et des moyens de détection (93) d'une bande de fréquence dans laquelle la densité spectrale de puissance du signal électroencéphalographique varie d'une valeur supérieure à un seuil prédéterminé, cette bande de fréquence correspondant à la bande de fréquence bêta spécifiée par les moyens de spécification (42).

7. Système de détection (9) électroencéphalographique selon la revendication 6, **caractérisé en ce que** les moyens de détermination (45) de la bande de fréquence bêta comportent en outre des moyens (87) de rétro-moyennage point à point du signal électroencéphalographique sur plusieurs intervalles de temps identiques, calés chacun sur des instants de déclenchement respiratoire ($t_0$) successifs, chaque intervalle s'étendant en partie en avance de l'instant de déclenchement respiratoire ($t_0$), et **en ce que** la carte temps-fréquence est obtenue à partir du signal électroencéphalographique moyenné.

8. Installation d'assistance ventilatoire (3), **caractérisée en ce qu'**elle comporte

   - une machine d'assistance ventilatoire (6) et
   - un système de détection électroencéphalographique d'un réglage inapproprié (9) selon l'une quelconque des revendications précédentes.

9. Installation d'assistance ventilatoire (3) selon la revendication 8, **caractérisée en ce qu'**elle comprend en outre une boucle de rétrocontrôle (78), propre à modifier le réglage de la machine d'assistance respiratoire (3) en fonction des mesures effectuées par le système de détection (9) électroencéphalographique d'un réglage inapproprié.

10. Procédé de détection d'un réglage inapproprié d'une machine d'assistance ventilatoire (6) utilisée sur un mammifère, comportant les étapes consistant à :

   - recevoir une mesure d'un signal électroencéphalographique en fonction du temps ;
   - déterminer, pour chaque cycle respiratoire, un instant de déclenchement respiratoire ($t_0$) ;
   - spécifier une bande de fréquence bêta comprise entre 15 et 30 Hz et de largeur comprise entre 5 et 10 Hz ;
   - traiter le signal électroencéphalographique mesuré, au fur et à mesure de son acquisition, dans la seule bande de fréquence bêta ; et
   - mettre en évidence, pour chaque cycle respiratoire, un réglage inapproprié de la machine d'assistance ventilatoire (6) à partir du signal électroencéphalographique traité dans la seule bande de fréquence bêta.

11. Produit programme d'ordinateur comportant des instructions qui, lorsque mis en oeuvre sur un calculateur associé à un électroencéphalographe (27), met en oeuvre le procédé selon la revendication 10.

**Patentansprüche**

1. System zur elektro-enzephalografischen Detektion (9) einer ungeeigneten Einstellung einer Beatmungsunterstützungsvorrichtung (6), die für einen Säuger verwendet wird, aufweisend:

   - einen Elektro-Enzephalograf (27), welcher in der Lage ist, in Abhängigkeit von der Zeit ein elektro-enzephalografisches Signal (s(t)) zu messen, welches für einen Atmungsvorgang repräsentativ ist, und
   - einen Eingang für den Empfang eines Atmungsauslösesignals, welches von dem elektro-enzephalografischen Signal (s(t)) verschieden ist und welches in der Lage ist, einen Atmungsauslösemoment ($t_0$) anzuzeigen,

   wobei das System zur Detektion (9) ferner aufweist:

   - Mittel zur Spezifikation (42) eines Betafrequenzbandes, welches im Bereich zwischen 15 und 30 Hertz liegt und eine Breite im Bereich zwischen 5 und 10 Hertz hat,
   - Mittel zum Verarbeiten (48) des gemessenen elektroenzephalografischen Signals, die dazu eingerichtet sind, das gemessene elektro-enzephalografischen Signal im Laufe seiner Erfassung, einzig in dem spezifizierten Betafrequenzband zu verarbeiten,
   - Mittel zur Identifizierung (51), für jeden Atmungszyklus, einer ungeeigneten Einstellung der Beatmungsunterstützungsvorrichtung (6) ausgehend von dem einzig in dem Betafrequenzband verarbeiteten elektro-enzephalografischen Signal.

2. System zur elektro-enzephalografischen Detektion (9) gemäß Anspruch 1, **dadurch gekennzeichnet, dass** die Mittel zum Identifizieren (51) einer ungeeigneten Einstellung dazu eingerichtet sind, für jeden Atmungszyklus, eine etwaige Desynchronisation des elektro-enzephalografischen Signals in dem spezifizierten Betafrequenzband zu identifizieren, wobei die besagte Desynchronisation dem Atmungsauslösemoment ($t_0$) vorausgeht.

3. System zur elektro-enzephalografischen Detektion (9) gemäß einem der Ansprüche 1 oder 2, **dadurch gekennzeichnet, dass** die Mittel zum Verarbeiten (48) ferner Berechnungsmittel aufweisen (57), die dazu eingerichtet sind, die momentane Leistung (P(t)) des elektroenzephalografischen Signals in dem Betafrequenzband zu berechnen, sowie Speichermittel (60), die dazu geeignet sind, die berechnete momentane Leistung (P(t)) zu speichern.

4. System zur elektro-enzephalografischen Erfassung (9) gemäß Anspruch 3, **dadurch gekennzeichnet, dass** die Berechnungsmittel (57) dazu geeignet sind, für jeden Atmungszyklus, ausgehend von der momentanen Leistung (P(t)), eine mittlere Leistung ($P_{mR}$) des elektroenzephalografischen Signals in dem Betafrequenzband in einem ersten Intervall ($I_R$) zu berechnen, dass die Mittel zum Identifizieren (51) Vergleichsmittel aufweisen, die dazu geeignet sind, die, in einem zweiten Intervall ($I_D$), welches sich vor dem Atmungsauslösemoment ($t_0$) erstreckt, berechnete momentane Leistung (P(t)) mit der in dem ersten Intervall berechneten mittleren Leistung ($P_{mR}$), zu vergleichen, wobei das erste Intervall ($I_R$) sich vor dem zweiten Intervall ($I_D$) erstreckt, und dass die Mittel zum Identifizieren (51) ferner Detektionsmittel (66) aufweisen, die dazu geeignet sind, für jeden Atmungszyklus, eine Diskrepanz zwischen der im ersten Intervall ($I_R$) berechneten mittleren Leistung ($P_{mR}$) und der im zweiten Intervall ($I_D$) berechneten momentanen Leistung (P(t)) zu erfassen.

5. System zur elektro-enzephalografischen Detektion (9) gemäß Anspruch 4, **dadurch gekennzeichnet, dass** die Mittel zum Indentifizieren (51) Mittel zum Auslösen (72) eines Indikators aufweisen, welche dazu geeignet sind, einen Indikator (75) auszulösen, wenn während einer vorbestimmten Anzahl an sukzessiven Atmungszyklen eine Diskrepanz auftritt.

6. System zur elektro-enzephalografischen Detektion (9) gemäß einem der vorhergehenden Ansprüche, **dadurch gekennzeichnet, dass** es ferner Mittel zur Bestimmung (45) des Betafrequenzbandes aufweist, welche den Mitteln zur Spezifikation (42) des Betafrequenzbandes vorgeschaltet sind, wobei die Mittel zur Bestimmung (45) des Betafrequenzbandes Mittel zur Erstellung einer Zeit-Frequenz-Karte (90) des von dem Elektro-Enzephalografen (27) gemessenen elektroenzephalografischen Signals aufweisen, wobei die besagte Zeit-Frequenz-Karte dazu geeignet ist, eine Entwicklung der spektralen Leistungsdichte des elektro-enzephalografischen Signals in Abhängigkeit von der Zeit und von der Frequenz des besagten Signals anzuzeigen, und Mittel zur Detektion (93) eines Frequenzbandes, in welchem die spektrale Leistungsdichte des elektro-enzephalografischen Signals einen Grenzwert um einen vorbestimmten Wert überschreitet, wobei dieses Frequenzband dem von den Mitteln zur Spezifikation (42) spezifizierten Betafrequenzband entspricht.

7. System zur elektro-enzephalografischen Detektion (9) gemäß Anspruch 6, **dadurch gekennzeichnet, dass** die Mittel zur Bestimmung (45) des Betafrequenzbandes ferner Mittel (87) zur Punkt für Punkt Rückmittelung des elektro-enzephalografischen Signals über mehrere identische Zeitintervalle aufweisen, wobei die Zeitintervalle jeweils aufeinanderfolgenden Atmungsauslösemomenten ($t_0$) zugeordnet sind und jedes Intervall sich teilweise vor dem zugeordneten Atmungsauslösemoment ($t_0$) erstreckt, und dass die Zeit-Frequenz-Karte ausgehend von diesem mittleren elektro-enzephalografischen Signal erstellt wird.

8. Beatmungsunterstützungseinrichtung (3), **dadurch gekennzeichnet, dass** sie aufweist

 - eine Beatmungsunterstützungsvorrichtung (6) und
 - ein System zur elektro-enzephalografischen Detektion einer ungeeigneten Einstellung (9) gemäß irgendeinem der vorhergehenden Ansprüche.

9. Beatmungsunterstützungseinrichtung (3) gemäß Anspruch 8, **dadurch gekennzeichnet, dass** sie ferner ausweist eine Rückführschleife (78), welche dazu geeignet ist, die Einstellung der Beatmungsunterstützungsvorrichtung (3) zu ändern in Abhängigkeit von den Messungen, die durch das System zur elektro-enzephalografischen Detektion (9) einer ungeeigneten Einstellung durchgeführt werden.

10. Verfahren zur Detektion einer ungeeigneten Einstellung einer Beatmungsunterstützungsvorrichtung (6), welche für einen Säuger verwendet wird, aufweisend die Schritte:

 - Erhalten einer Messung eines elektro-enzephalografischen Signals in Abhängigkeit von der Zeit
 - Bestimmen eines Atmungsauslösemoments ($t_0$) für jeden Atmungszyklus,
 - Spezifizieren eines Betafrequenzbandes im Bereich zwischen 15 bis 30 Hertz und einer Breite im Bereich zwischen 5 und 10 Hertz,
 - Verarbeiten des gemessenen elektro-enzephalografischen Signals im Laufe seiner Erfassung, einzig in dem Betafrequenzband, und
 - Identifizieren einer ungeeigneten Einstellung der Atmungsunterstützungsvorrichtung (6), für jeden Atmungszyklus, ausgehend von dem einzig in dem Betafrequenzband verarbeiteten elektro-enzephalografischen Signal.

11. Computerprogrammprodukt, welches Anweisungen umfasst, welche, wenn sie auf einem Computer, der mit einem Elektro-Enzephalografen (27) verbunden ist, durchgeführt werden, das Verfahren gemäß Anspruch 10 durchführen.

**Claims**

1. A system (9) for electroencephalographic detection of an improper adjustment of a ventilatory support machine (6) used on a mammal, comprising:

   - an electroencephalograph (27), capable of measuring, as a function of time, an electroencephalographic signal (s(t)) representative of a breathing process; and
   - an input for receiving a respiratory initiation signal, different from the electroencephalographic signal (s(t)), capable of indicating a respiratory initiation time ($t_0$);

   the detection system (9) further including

   - means (42) for specifying a beta frequency band comprised between 15 and 30 Hz and with a width comprised between 5 and 10 Hz;
   - means (48) for processing the measured electroencephalographic signal, configured for processing the measured electroencephalographic signal as it is being acquired, in the sole specified beta frequency band;
   - means for identifying (51), for each breathing cycle, an improper adjustment of the ventilatory support machine (6) from the electroencephalographic signal processed in the sole beta frequency band.

2. The electroencephalographic detection system (9) according to claim 1, **characterized in that** the means (51) for identifying an improper adjustment are configured for identifying, for each breathing cycle, a possible desynchronization of the electroencephalographic signal in the specified beta frequency band, said desynchronization preceding the respiratory initiation time ($t_0$).

3. The electroencephalographic detection system (9) according to one of claims 1 or 2, **characterized in that** the processing means (48) further comprise computation means (57), configured for calculating the instantaneous power (P(t)) of the electroencephalographic signal in the sole beta frequency band, as well as storage means (60) capable of storing the calculated instantaneous power (P(t)).

4. The electroencephalographic detection system (9) according to claim 3, **characterized in that** the computation means (57) are capable of calculating, for each breathing cycle, from the instantaneous power (P(t)), an average power ($P_{mR}$) of the electroencephalographic signal in the sole beta frequency band over a first interval ($I_R$), **in that** the identification means (51) comprise means (63) for comparing the instantaneous power (P(t)) calculated in a second interval ($I_D$) extending in advance of the respiratory initiation time ($t_0$) with the average power ($P_{mR}$) calculated over the first interval, the first interval ($I_R$) extending in advance of the second interval ($I_D$), and **in that** the identification means (51) further comprise detection means (66), capable of detecting, for each breathing cycle, a deviation between the average power ($P_{mR}$) calculated in the first interval ($I_R$) and the instantaneous power (P(t)) calculated in the second interval ($I_D$),

5. The electroencephalographic detection system (9) according to claim 4, **characterized in that** the identification means (51) comprise means (72) for triggering an indicator, capable of triggering an indicator (75) if a deviation is detected for a predetermined number of successive breathing cycles.

6. The electroencephalographic detection system (9) according to any of the preceding claims, **characterized in that** it further comprises, at the input of the means for specifying the beta frequency band (42), means (45) for determining the beta frequency band, said means (45) for determining the beta frequency band including means for establishing a time-frequency map (90) of the electroencephalographic signal measured by the electroencephalograph (27), said time-frequency map being capable of indicating the change in the power spectral density of the electroencephalographic signal as a function of time and of the frequency of said signal and means (93) for detecting a frequency band in which the power spectral density of the electroencephalographic signal varies by a value greater than a predetermined threshold, this frequency band corresponding to the beta frequency band specified by the specification means (42).

7. The electroencephalographic detection system (9) according to claim 6, **characterized in that** the means (45) for determining the beta frequency band further comprise means (87) for back-averaging point by point the electroencephalographic signal over several identical time intervals, being each set on successive respiratory initiation times ($t_0$), each interval partly extending in advance of the respiratory initiation time ($t_0$), and **in that** the time-frequency map is obtained from the averaged electroencephalographic signal.

**8.** A ventilatory support installation (3), **characterized in that** it includes

> - a ventilatory support machine (6) and
> - a system for electroencephalographic detection of an improper adjustment (9) according to any of the preceding claims.

**9.** The ventilatory support installation (3) according to claim 8, **characterized in that** it further comprises a feedback control loop (78) capable of modifying the adjustment of the ventilatory support machine (3) according to the measurements carried out by the system (9) for electroencephalographic detection of an improper adjustment.

**10.** A method for detecting an improper adjustment of a ventilatory support machine (6) used on a mammal, including the steps:

> - receiving a measurement of an electroencephalographic signal as a function of time;
> - determining, for each breathing cycle, a respiratory initiation time ($t_0$);
> - specifying a beta frequency band comprised between 15 and 30 Hz and with a width comprised between 5 and 10 Hz;
> - processing the measured electroencephalographic signal, as it is being acquired, in the sole beta frequency band; and
> - identifying, for each breathing cycle, an improper adjustment of the ventilatory support machine (6) from the electroencephalographic signal processed in the sole beta frequency band.

**11.** A computer program product including instructions which, when applied on a computer associated with an electroencephalograph (27) carries out the method according to claim 10.

FIG.1

$s(t)$   (1)

$s'(t)$   (2)

$P(t)$   (3)

$R'(t)$   (4)

$d_R$   $d_D$   $t_0$

$I_R$   $I_D$

## FIG.2

**FIG.3**

FIG.4

## FIG.5

EP 2 590 701 B1

<u>FIG.6</u>

EP 2 590 701 B1

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2903314 A